# EUROPEAN PATENT APPLICATION

(11) **EP 3 248 651 A1**
(43) Date of publication of application: **29.11.2017**
(21) Application number: 17173232.4
(22) Date of filing: 29.05.2017
(51) Int. Cl.: A61N 5/06, A61B 18/22

(54) **SOURCE-INSENSITIVE CYLINDRICAL LIGHT DIFFUSER AND VISUAL INDICATOR SYSTEM FOR PHOTOTHERAPY**

(30) Priority: 27.05.2016 US 201615167748
(71) Applicant: Lasertel Inc., 85743 AZ Arizona, Tucson (US)
(72) Inventor: Moor, Robert N., Tucson, AZ Arizona 85719 (US); Jean, Justin K., Tucson, AZ Arizona 85730 (US); Lohrenz, Ryan, Vail, AZ Arizona 85641 (US)
(74) Representative: Bittner, Bernhard

(57) **Abstract**

A diffuser tip assembly is disclosed for generation of uniform cylindrical illumination from a fiber delivered source. Light propagating into the diffuser tip is initially mixed by a spatial overlap of reflections within a waveguide, reducing the sensitivity of illumination uniformity to the modal structure of fiber delivered light. The waveguide output propagates at least two passes through a reflective cavity having transmissive, light-diffusive walls, enabling highly uniform output. The diffuser tip can be configured to use low-absorbing materials for high power applications. In addition, the method of using visible light as an indicator for diffuser output is described. The combination of uniformity, low heat generation, and a visual indicator are intended to promote safety in a phototherapy procedure.

## Description

### FIELD OF THE INVENTION

The present invention is generally related to phototherapy devices and more particularly is related to a diffuser tip for providing cylindrical illumination for the elimination of bacteria in an orifice.

### BACKGROUND OF THE INVENTION

Phototherapy is becoming an increasingly commonplace treatment method for various medical conditions. In a phototherapy process, a treatment area is exposed to specific frequencies of light with controlled distribution and duration. Phototherapy can be used for the treatment of various skin disorders, sleep and mood affective disorders, and to counteract the development of bacteria, fungi, or other biological contaminants. In a particular instance, phototherapy may be used with antibiotics to eliminate biological contaminants within the human body, such as bacterial infections.

Laser-based phototherapy allows for high intensity light sources to be delivered to a target area by means of optical fiber, and is especially practical for application internal to the body. A specialized application device may be used to tailor the distribution of light onto the treatment area. Cylindrical diffusers are a common form of application tip, and can in some instances be produced from modification of the delivery fiber.

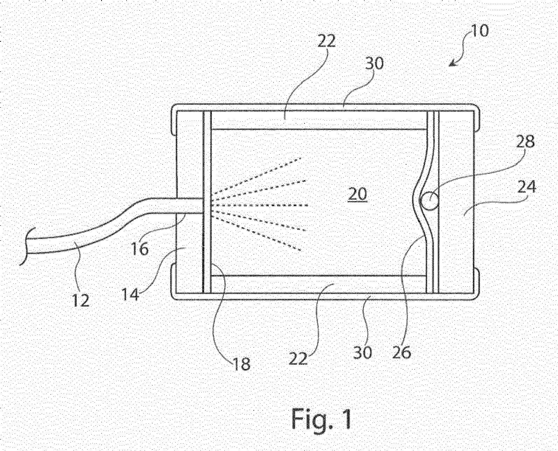

Fig 1 is a schematic diagram of a cylindrical diffuser 10 in accordance with prior art, for treatment applications requiring relatively large diameter compared to the core diameter of the delivery fiber 12. The delivery fiber 12 enters the cylindrical diffuser through an opening 16 in a proximal end 14 of the cylindrical diffuser. The fiber is terminated into an empty cavity 20. A specular reflective film 26 such as metallized mylar film is located interior to a distal end 24. A small sphere 28 or other feature may be used to impart a convex shape to the reflective film. A diffuse reflecting material 18 is applied to the interior or the proximal end 14. The diffuser body 22 is made from a polytetrafluoroethylene (PTFE) tube and held to the proximal end 14 and distal end 24 by a clear heat shrink plastic 30. In a simplified model of use, light from the delivery fiber 12 propagates through the cavity 20 toward the specular reflective film 26. A portion of the light is reflected to the diffuse reflecting material 18 and eventually all unabsorbed light is directed to transmit through the diffuser body 22 to exit the cylindrical diffuser 10 to the patient's body.

The diffuser of Fig 1 has been used with some success, but also has a number of shortcomings. Undesired heat is created by residual absorption of some of the materials. Emitted light is often unevenly distributed due in part to sensitivity to the angular light distribution from the delivery fiber 12. The build-up of heat and the uneven distribution of light from the diffuser tip 10 can result in damage to healthy tissues in the patient's body due to overheating of some areas and under-treatment of others.

Thus, a heretofore unaddressed need exists in the industry to address the aforementioned deficiencies and inadequacies.

### SUMMARY OF THE INVENTION

Embodiments of the present invention provide a system and method for a diffuser tip for an optical delivery apparatus. Briefly described, in architecture, one embodiment of the system, among others, can be implemented as follows.

The diffuser tip comprises a cavity formed along a diffuser axis, between a proximal end surface and a distal end surface, and configured to receive light from the distal end of an optical delivery apparatus, typically an optical fiber. At least the distal end surface of the diffuser tip cavity is reflective. The cavity interior may be separated into a homogenizer region and an emission region, configured such that light received from the fiber distal end is homogenized and eventually emitted from the diffuser tip in a direction transverse or oblique to the diffuser axis. A portion of the light may be lost or absorbed.

Light from the fiber distal end is received through a bore in the proximal end surface of the diffuser. The distal end of the fiber may be allowed to protrude minimally into the cavity before termination. In one embodiment, the fiber distal end face may be cleaved or polished flat. In another embodiment, the fiber distal end face may be roughly ground.

One or both of the diffuser tip end surfaces may be a surface subsection of a larger component or assembly, including an assembly performing at least one function beyond the scope of this disclosure. In one configuration, the proximal end surface may be a surface subsection of a larger diffuser base, with features for handling, mounting and fiber strain relief.

In some embodiments, the diffuser tip may be encased in a transparent tube, such as a tube fabricated from glass or transparent plastic material. The transparent tube may be attached to the diffuser base and to a distal end to form a bonded structure. Components within the transparent tube may be loosely constrained within the bonded structure.

A waveguide homogenizer reduces the sensitivity of the diffuser output distribution to the distribution of light received from the delivery fiber. In its simplest form, the homogenizer comprises a distance along the cavity axis in which light is confined laterally by reflective walls, with sufficient length such that some portion of the system light is reflected and overlapped spatially. In one embodiment, it comprises a cylindrical waveguide within a lower refractive-index bulk material.

A diffusing surface (or volume) may be added to broaden the angles of the light being input into the proximal end of waveguide. Alternatively, the waveguide may be tapered to achieve a similar effect. In doing so, the length of the waveguide necessary to achieve multiple orders of successive reflections may be reduced. However, attention must be paid to the length of the intended emission region, as steeper angles of light will out-couple more quickly, potentially reducing the axial uniformity of the output.

The diffusing surface may be configured as a surface of the cylinder waveguide or the distal end face of the fiber. A volume of bulk scattering material may be used in place of a diffusing surface. In one embodiment, a disk with a textured surface is bonded to the proximal end of the cylinder waveguide.

The emission region comprises a cylindrical volume encompassed laterally by a diffusing, cylindrical surface or layer. In on embodiment, the emission region may comprise a hollowed cylindrical volume within a tube of the diffusing material, such as PTFE.

A reflective surface prevents light transmission through a distal end. The reflective surface may be a utilized a reflective coating or may be made from a bulk reflective material. In one embodiment, the reflective surface is a broadband dielectric coating deposited onto a glass disk substrate.

A diffusing material may be used at the distal end to increase light output near the distal end of the emission region. In one embodiment, the reflective surface is itself diffuse. In another embodiment, light transmits through a diffusing surface or material prior to reflection. In one embodiment, a thin PTFE film covers the reflective surface. The reflective surface is chosen for high reflectivity over the full spectral range of the system light.

The delivery fiber supplies specific treatment frequencies of light, which may include light outside of the visible frequency range, such as infrared light. In one aspect of the disclosure, a quantity of visible light is additionally supplied by the delivery fiber. The visual indicator may be used to indicate the distribution of treatment light from the emission region. Materials and coatings used by the diffuser optics may be tailored to function without sensitivity to supplied frequencies of light.

A removable protective cover, including a disposable cover, may be placed over the diffuser optics and attached to the diffuser. A disposable cover may comprise an optically-transmissive plastic body. The disposable cover may have features for attachment to patient body. In one embodiment, a plastic clip is connected onto, or a part of, the disposable cover, wherein the clip aids retention of the diffuser emission region within an orifice of the patient.

To prevent heating, embodiments of the disclosed apparatus should minimize the absorption of light by materials within the diffuser tip. For this reason, embodiments utilizing total internal reflection (TIR) and/or dielectric coating reflectors are preferred over metallic reflectors.

The present invention also can be viewed as providing an optical delivery apparatus comprising: an optical transmission system including an optical waveguide; having a proximal end and a distal end; said optical transmission system being configured to receive system light from a source into its proximal end and to deliver system light to its distal end; a diffuser tip, comprising a cylindrical cavity formed between a proximal end and a distal end, wherein said cavity is configured to receive system light from the optical waveguide in a generally axial direction; said distal end having a distal end surface configured to reflect at least some incident system light, and said cavity being divisible into functional segments along its axis, including a waveguide homogenizer segment, wherein at least a portion of system light is substantially laterally confined by at least one reflective barrier, and said waveguide homogenizer segment is configured to facilitate spatial overlap; said cavity further containing at least one emission segment, located distal to the waveguide homogenizer segment, said emission segment comprising a volume which is substantially transparent to system light, and being encompassed radially by at least one interface, wherein said at least one interface is at least partially transmissive to system light, such that at least a portion of said system light may emitted from the cavity of the diffuser tip; and, wherein at least one said interface is configured to angularly diffuse at least a portion of incident system light.

In one aspect the at least one waveguide homogenizer segment comprises at least one substantially transparent volume having a proximal end and a distal end, extending along the cavity axis, and having at least one transverse surface which is substantially reflective to at least a portion of system light; said volume being configured such that at least a portion of system light may be coupled into a proximal or distal end of said waveguide, and emitted from said distal end or proximal end of said waveguide; said volume being configured such that at least a portion of said system light may be substantially confined by the transverse surfaces; said volume having sufficient axial extent for at least a portion of system light to be reflected by said transverse surfaces. In such embodiment, a distal face of the transmission system is textured to angularly diffuse system light prior to the coupling of at least some of said system light into the waveguide.

In such aspect the waveguide homogenizer segment preferably comprises a core formed by a volume of material, said volume being substantially transparent to system light and surrounded radially or transversely by a volume or layer having lower refractive index to system light, such that at least a portion of system light is confined within the waveguide by total internal reflection. Also in such embodiment, the wavelength has a proximal face textured to angularly diffuse incident light.

In another aspect the emission segment preferably comprises a volume within the cavity of the diffuser tip, and extending along the cavity axis, encircled radially by a section of tube, where said tube is formed from a material or combination of materials having properties to substantially diffuse at least a portion of said system light. In such aspect, the emission segment preferably comprises a volume within the cavity of the diffuser tip, and extending along the cavity axis, encircled radially by a section of tube; wherein said tube is constructed from a material or combination of materials having properties to substantially diffuse at least a portion of said system light, and wherein the tube is extended into at least one waveguide homogenizer segment, and functions as a lower refractive index layer for said waveguide.

In a preferred aspect the tube encompassing the emission region is formed from PTFE.

In another aspect, the optical delivery apparatus has a transmissive surface or volume, located in the cavity between the proximal end and the waveguide, and configured to angularly diffuse light prior to coupling of at least some system light into the waveguide. In such aspect, the optical delivery apparatus preferably has at least one textured face, configured to angularly diffuse said system light, located in the cavity between the proximal end of the cavity and the waveguide. In such aspect, at least one lens preferably is used to collimate or partially collimate radiation emissions from the distal end of the transmission system, prior to propagation of the light into an angular diffuser of the waveguide homogenizer segment.

In still other aspects, the proximal end of the wavelength is configured to reflect at least a portion of said incident system light, reflected or scattered from a more distal portion of the cavity and/or the distal end of the cavity is configured to diffusely reflect at least a portion of said incident system light.

In yet another aspect, the distal end of the cavity is configured to specularly reflect at least a portion of said incident system light, and wherein a transmissive volume is placed within the cavity, distal an emission segment and proximal the distal end of the cavity, and configured to angularly diffuse transmitted light.

In yet other aspects, the distal end of the transmission system protrudes into the diffuser tip through an opening in the cavity proximal surface; the cavity of the diffuser tip optionally is encompassed radially, at least in part along its axis, by one or more tubes or housings; the proximal surface and/or distal surface of the diffuser tip optionally is a portion of a volume having features for handling or mounting the diffuser; and the diffuser tip optionally is encased in a removable sheath, formed of a material which is at least partially transmissive to system light.

The present invention also provides a medical or cosmetic phototherapydevice, comprising a light source for generating system light at frequencies intended for treatment, and an optical delivery apparatus as above described. In such embodiment a portion of said system light preferably is at a visible frequency.

In another embodiment the optical delivery apparatus cavity preferably contains an extra emission segment which functions as a visual indicator when at least one other emission segment is obscured during a treatment process.

The present invention also provides optical therapy device for providing therapeutic light for the elimination of bacteria in a human nostril, comprising: an optical delivery apparatus as above described; and a light source in optical communication with the proximal end of the optical transmission system

The present invention can also be viewed as providing safe methods for eliminating bacteria from a human orifice. In this regard, one embodiment of such a method, among others, can be broadly summarized by the following steps: positioning of diffuser emission region within a patient orifice, and emission of a quantity of treatment light and a quantity of visible indicator light from the diffuser.

Other systems, methods, features, and advantages of the present invention will be or become apparent to one with skill in the art upon examination of the following drawings and detailed description. It is intended that all such additional systems, methods, features, and advantages be included within this description, be within the scope of the present invention, and be protected by the accompanying claims.

### BRIEF DESCRIPTION OF THE DRAWINGS

Many aspects of the invention can be better understood with reference to the following drawings. The components in the drawings are not necessarily to scale, emphasis instead being placed upon clearly illustrating the principles of the present invention. Moreover, in the drawings, like reference numerals designate corresponding parts throughout the several views.
FIG. 1 is a schematic diagram of a diffuser tip, in accordance with the prior art.
FIG. 2 is a cross-sectional view illustration of a diffuser tip for an optical delivery apparatus, in accordance with a first exemplary embodiment of the subject disclosure.
FIG. 3 is a simplified block diagram of an optical delivery system in accordance with an exemplary embodiment of the subject disclosure.
FIG. 4 is a partial cross-sectional illustration of a diffuser tip for an optical delivery apparatus positioned within an orifice, in accordance with the first exemplary embodiment of the subject disclosure.
FIG. 5 is a flowchart illustrating a method of eliminating bacteria from a human orifice, in accordance with the first exemplary embodiment of the subject disclosure.

### DETAILED DESCRIPTION

As used herein, a quantity of light may be said to propagate along any conceptually useful direction 'generally' if a representative light propagation vector has a greater magnitude component along said direction than along any orthogonal direction, said representative vector consisting of the weighted average of all propagation vectors for said quantity of light. By way of example, but not limitation, a quantity of light may be said to propagate along any direction 'generally' if a representative vector propagates approximately at said direction, within 45° but more typically within 5°.

The subject disclosure is directed to a cylindrical diffuser tip for an optical delivery system. The optical delivery system may attach to a laser system or other light source, which can have useful properties for medical treatment, as well as potential non-medical applications. As a primary example, the optical delivery system may be used in a medical process to eliminate bacteria within a nasal cavity. Other uses of the cylindrical diffuser apparatus and optical delivery system are anticipated.

FIG. 1 is a schematic diagram of a cylindrical diffuser, in accordance with prior art. A detailed description of FIG.1 is provided in the section Background of the Invention.

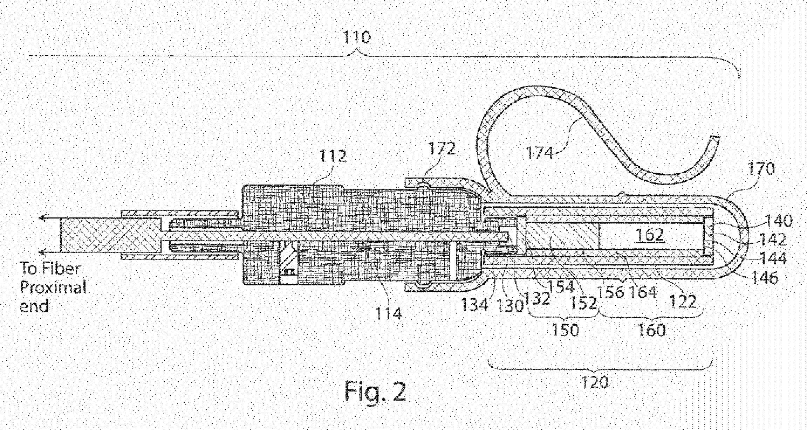

FIG. 2 is a cross-sectional view illustration of a diffuser tip 120 for an optical delivery system, in accordance with a first exemplary embodiment of the present disclosure. As is shown in FIG. 2, the diffuser tip 120 may attach to a base assembly 112 which may be constructed from a metal. The base assembly 112 may function as a handle or as an attachment body for a delivery fiber 114 and/or a disposable cover 170.

The diffuser tip 120 consists of a transparent tube 122 which may be constructed from glass or a plastic, such as transparent polycarbonate or acrylic. The transparent tube 122 may be situated spanning a proximal end 130 and a distal end 140, forming an enclosed volume. The transparent tube 122 may be attached or bonded to the proximal end 130 and distal end 140 structures.

As shown, the proximal end 130 is defined by a proximal end surface 134, which may be reflective, and which may also be subsection of the surface of the base assembly 112. The delivery fiber 114 may protrude a short distance through the proximal end 130 to its termination point at the fiber distal end 132.

A waveguide homogenizer assembly 150 is situated within the transparent tube 122 near the proximal end 130. The waveguide homogenizer segment 150 shown comprises a waveguide volume 152, laterally encased within a reflective boundary 156, and a planar diffuser 154. The waveguide 152 may be constructed from a length of polished transparent glass. A reflective boundary 156 is formed by total internal reflection (TIR) at the interface between the glass and surrounding lower refractive index material, which may be constructed from PTFE and may be produced as an extension of the light diffusing tube 164 used in the emission region 160. The diffuser 154 is fabricated as a textured surface on a glass disk, attached to the waveguide 152, and held at its edges between the proximal end 130 and the low refractive index tube serving as a waveguide cladding 164. The low refractive index material 156.

The emission segment 160 may comprise a cylindrical volume 162 laterally encased within a light diffusing boundary 164. The boundary 164 may be constructed as a PTFE tube, and the cylindrical volume 162 may comprise the interior airspace.

A reflective surface 144 is located near a distal end 140. The reflective surface 144 may be fabricated as a dielectric coating deposited onto the surface of a solid disk 142, which may be constructed from glass. A light diffuser 146 may be used if the reflective surface is specular. The planar diffuser may be formed from a thin layer of PTFE, held in compression between the distal end face 144 and the PTFE tube 164.

A disposable cover 170 may be placed over and around the diffuser tip 120. The disposable cover 170 may attach to a base assembly 112 using mounting features 172, and may have features to attach to a patient 174. The disposable cover 170 may be constructed from a plastic with negligible absorption of supplied system light.

In use, light may enter the diffuser tip 120 interior to the proximal end 130 after emission from the fiber distal end 132. The light may be directed forward, expanding along an axis formed between the proximal end 130 and the distal end 140, to first intersect the planar diffuser 154. The diffuser 154 mixes and broadens the propagation angles of the system light. The light then propagates into and through the waveguide 152, and is mixed spatially by overlapping orders of reflection against the waveguide walls. The light then exits the waveguide 152 into the cavity 162 of the emission region 160, wherein a portion of the light is distributed to the PTFE tube 164. This portion is most-heavily comprised of light with higher propagation angles relative to the propagation axis. A second portion, mostly light with lower propagation angles, is directed toward the distal end 140 through a diffuser 146 and to the reflective surface 144. The reflective surface 144 returns light to the emission segment 160, with higher propagation angles, whereupon it may be directed to the PTFE tube 164.

A portion of the reflected light may return through the waveguide 152 to the proximal end surface 134, and may again be reflected back. If the waveguide 142 does not fully extend to the proximal end surface 134, a small portion of light may be allowed to leak from the diffuser tip 120 near the proximal end 130.

Much of the light which intersects the PTFE tube 164 is directed to its outer surface, and emitted through the transparent tube 122 and disposable cover 170. Some portion of light may be reflected by the PTFE tube 164 back into the underlying cavity 162. The process of mixing and re-mixing the light paths provides for a smooth distribution of light down the length of the emission region 160.

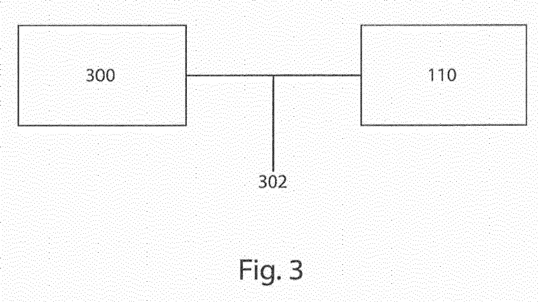

FIG. 3 is a simplified block diagram of an optical delivery system in accordance with the subject disclosure. The system includes a light source 300 connected via an optical waveguide 302 to a diffuser tip 110 as illustrated in FIG. 2.

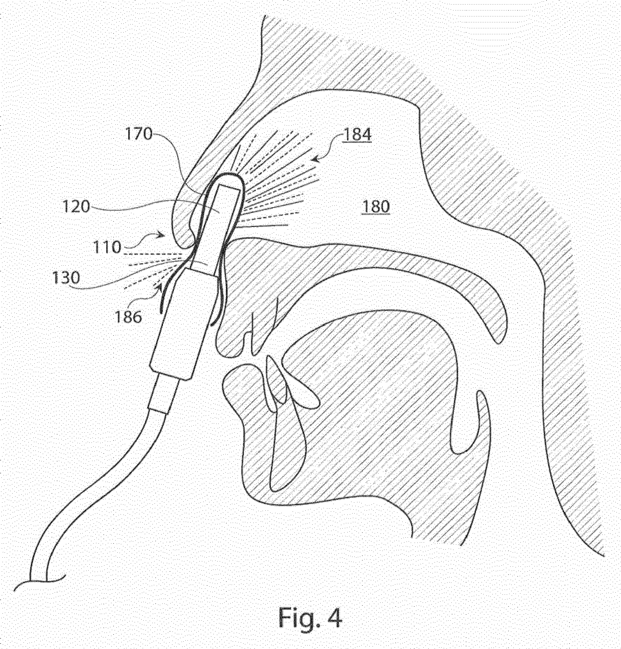

FIG. 4 is a partial cross-sectional illustration of an optical delivery apparatus 110 for a phototherapy application positioned within an orifice, in accordance with the first exemplary embodiment of the subject disclosure. As is shown, the diffuser tip 120 may be positioned to provide treatment to an orifice of a human being, such as within a patient's nasal cavity. In this example, the diffuser tip 120 and the overlying portion of the disposable cover 170, extend into the patient's nasal cavity 180 to a degree or position selected by a medical professional. Light that is supplied to the delivery fiber 114 may be comprised of a mixture of light intended for treatment, commonly IR, and visible indicator light. In use, the light is routed from the delivery fiber 114, through the diffuser tip 120 and disposable cover 170 and into the nasal cavity 180. Light from the emission region is indicated as 184 in FIG. 4. A small portion of light may be permitted to leak out of the diffuser tip 120 near the proximal end 130, so that visible indicator light may be monitored by a medical professional (as indicated by light exiting nostril, 186 in FIG. 4). After treatment, the disposable cover may be removed and discarded.

It is noted that the diffuser tip 120 as described within this disclosure may offer significant benefits over the conventional art. For one, the use of the diffuser tip may reduce or eliminate heating problems commonly found in conventional devices. Further, the diffuser tip is not sensitive to the angular distribution of the light transported by the delivery fiber, and can be made to provide consistent, uniform illumination. The diffuser tip can also be constructed to be spectrally insensitive over wide bands for applications requiring many frequencies of light. The spectral insensitivity of the diffuser tip allows for use of a visible light to indicate the emission profile to an operator. It is also noted that the uniform illumination offered by the diffuser apparatus can be used for other applications, such as industrial applications.

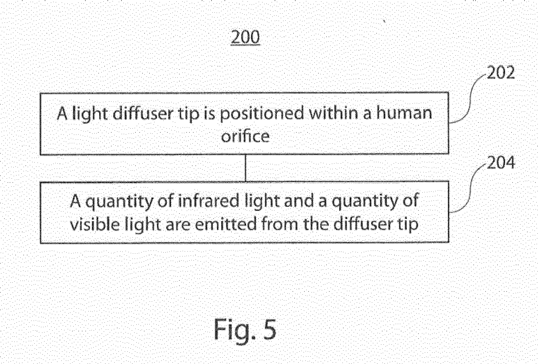

FIG. 5 is a flowchart 200, illustrating a method of eliminating bacteria from a human orifice, in accordance with the first exemplary embodiment of the subject disclosure. It should be noted that any process descriptions or blocks in flow charts should be understood as representing modules, segments, or steps that include one or more instructions for implementing specific logical functions in the process, and alternate implementations are included within the scope of the present invention in which functions may be executed out of order from that shown or discussed, including substantially concurrently, or in reverse order, depending on the functionality involved, as would be understood by those reasonably skilled in the art of the present invention.

As is shown by block 202, a diffuser apparatus may be positioned within a human orifice. A quantity of treatment light and a quantity of visible light may be emitted from the diffuser apparatus (block 204). The method may further include any number of additional steps, processes, and functions, including any disclosed relative to any other figure of this disclosure.

It should be emphasized that the above-described embodiments of the present invention, particularly, any "preferred" embodiments, are merely possible examples of implementations, provided for a clear understanding of the principles of the invention. Many variations and modifications may be made to the above-described embodiment(s) of the invention without departing substantially from the spirit and principles of the invention. All such modifications and variations are intended to be included herein within the scope of this disclosure and the present invention and protected by the following claims.

## Claims

1. An optical delivery apparatus **characterized by** comprising:
an optical transmission system including an optical waveguide having a proximal end and a distal end; said optical transmission system being configured to receive system light from a light source into its proximal end and to deliver system light to its distal end;
a diffuser tip, comprising a cylindrical cavity formed between a proximal end and a distal end, wherein said cavity is configured to receive system light from the optical waveguide in a generally axial direction; said distal end having a distal end surface configured to reflect at least some incident system light, and said cavity being divisible into functional segments along its axis, including a waveguide homogenizer segment, wherein at least a portion of system light is substantially laterally confined by at least one reflective barrier, and said waveguide homogenizer segment is configured to facilitate spatial overlap;
said cavity further containing at least one emission segment, located distal to the waveguide homogenizer segment, said emission segment comprising a volume which is substantially transparent to system light, and being encompassed radially by at least one interface, wherein said at least one interface is at least partially transmissive to system light, such that at least a portion of said system light may emitted from the cavity of the diffuser tip, and,
wherein at least one said interface is configured to angularly diffuse at least a portion of incident system light.

2. The apparatus of claim 1 **characterized in that** said at least one waveguide homogenizer segment comprises at least one substantially transparent volume having a proximal end and a distal end, extending along the cavity axis, and having at least one transverse surface which is substantially reflective to at least a portion of system light;
said volume being configured such that at least a portion of system light may be coupled into a proximal or distal end of said waveguide, and emitted from said distal end or proximal end of said waveguide;
said volume being configured such that at least a portion of said system light may be substantially confined by the transverse surfaces; and
said volume having sufficient axial extent for at least a portion of system light to be reflected by said transverse surfaces.

3. The apparatus of claim 2 **characterized in that** the waveguide homogenizer segment comprises a core formed by a volume of material, said volume being substantially transparent to system light and surrounded radially or transversely by a volume or layer having lower refractive index to system light, such that at least a portion of system light is confined within the waveguide by total internal reflection.

4. The apparatus of claim 2 **characterized in that** said emission segment comprises a volume within the cavity of the diffuser tip, and extending along the cavity axis, encircled radially by a section of tube, where said tube is formed from a material or combination of materials having properties to substantially diffuse at least a portion of said system light, wherein said emission segment preferably comprises a volume within the cavity of the diffuser tip, extending along the cavity axis, encircled radially by a section of tube;
wherein said tube is constructed from a material or combination of materials having properties to substantially diffuse at least a portion of said system light; and
wherein the tube is extended into at least one waveguide homogenizer segment, and functions as a lower refractive index layer for said waveguide, and wherein the tube encompassing the emission region preferably is formed from PTFE.

5. The apparatus of claim 2 **characterized by** having a transmissive surface or volume, located in the cavity between the proximal end and the waveguide, and configured to angularly diffuse light prior to coupling of at least some system light into the waveguide, and optionally having at least one textured face, configured to angularly diffuse said system light, located in the cavity between the proximal end of the cavity and the waveguide.

6. The apparatus of claim 3 **characterized in that** the waveguide has a proximal face textured to angularly diffuse incident light.

7. The apparatus of claim 2 **characterized in that** a distal face of the transmission system is textured to angularly diffuse system light prior to the coupling of at least some of said system light into the waveguide.

8. The apparatus of claim 5 **characterized in that** at least one lens is used to collimate or partially collimate radiation emissions from the distal end of the transmission system, prior to propagation of the light into an angular diffuser of the waveguide homogenizer segment.

9. The apparatus of claim 1 **characterized by** one or more of the following features:
(a) the proximal end is configured to reflect at least a portion of said incident system light, reflected or scattered from a more distal portion of the cavity;
(b) wherein the distal end of the cavity is configured to diffusely reflect at least a portion of said incident system light;
(c) wherein the distal end of the cavity is configured to specularly reflect at least a portion of said incident system light, and wherein a transmissive volume is placed within the cavity, distal an emission segment and proximal the distal end of the cavity, and configured to angularly diffuse transmitted light;
(d) wherein the distal end of the transmission system protrudes into the diffuser tip through an opening in the cavity proximal surface;
(e) wherein the cavity of the diffuser tip is encompassed radially, at least in part along its axis, by one or more tubes or housings, wherein the optical delivery apparatus cavity preferably contains an extra emission segment which functions as a visual indicator when at least one other emission segment is obscured during a treatment process;
(f) wherein the proximal surface and/or distal surface of the diffuser tip is a portion of a volume having features for handling or mounting the diffuser; and
(g) wherein the diffuser tip is encased in a removable sheath, formed of a material which is at least partially transmissive to system light.

10. A medical or cosmetic phototherapydevice, **characterized by** comprising a light source for generating system light at frequencies intended for treatment, and an optical delivery apparatus as claimed in any of claims 1-9.

11. The apparatus of claim 10, **characterized in that** a portion of said system light is at a visible frequency.

12. An optical therapy device for providing therapeutic light for the elimination of bacteria in a human nostril, **characterized by**:
an optical delivery apparatus as claimed in any of claims 1-9; and
a light source in optical communication with the proximal end of the optical transmission system.
